Europäisches Patentamt

European Patent Office

Office européen des brevets

(11) Publication number: **0 093 497**
**A2**

(12) **EUROPEAN PATENT APPLICATION**

(21) Application number: **83301557.1**

(22) Date of filing: **21.03.83**

(51) Int. Cl.³: **A 61 K 31/415**
**C 07 D 235/32**

(30) Priority: **30.04.82 GB 8212766**

(43) Date of publication of application:
**09.11.83 Bulletin 83/45**

(84) Designated Contracting States:
**BE CH DE FR GB IT LI LU NL SE**

(71) Applicant: **SMITH KLINE & FRENCH LABORATORIES LIMITED**
**Mundells**
**Welwyn Garden City Hertfordshire, AL7 1EY(GB)**

(72) Inventor: **Dickson, Brian**
**427 Daventry Road**
**Berwyn Philadelphia(US)**

(74) Representative: **Johnston, Walter Paul et al,**
**Patent Department SMITH KLINE & FRENCH**
**LABORATORIES LTD Mundells**
**Welwyn Garden City Hertfordshire AL7 1EY(GB)**

(54) Anthelmintic treatment.

(57) The invention relates to albendazole for use by oral administration in the treatment of hydatid disease in man.

EP 0 093 497 A2

- 1 -

## ANTHELMINTIC TREATMENT

This invention relates to albendazole (methyl 5-propylthio-2-benzimidazolecarbamate) for use in the treatment of hydatid disease in man.

Hydatid disease is a parasitic infestation of mammals caused by the organism Echinococcus. The causative organism is found in sheep and cattle, dogs, and man (where the adult worm forms a cyst). The organism is picked up by ingestion. It is transmitted from the intestine to the blood, then to the liver and subsequently to the whole body. In man these cysts can be life-threatening.

To date, the only effective way in which hydatid cysts could be treated in man is with surgery. However, the operation is difficult to perform because it carries the risk that the cyst might be ruptured. If it is ruptured, its contents (which includes the nuclei of other cysts) disperse throughout the body setting up secondary cysts. Dispersal of the liquid content of the cyst can also cause anaphylaxis and death.

There is no known effective chemotherapeutic treatment for this condition. Many anthelmintics, particularly benzimidazole anthelmintics, have been tried for the treatment of this disease but to our knowledge none have been successful.

We have now found that it is possible to treat hydatid disease in man by oral administration of albendazole. Albendazole is a known anthelmintic agent. It is disclosed in British Patent Specification No. 1,464,326.

According to the present invention there is provided albendazole for use by oral administration in the treatment of hydatid disease in man.

The dose and the term of treatment can be set having regard to the age and weight of the patient and the severity of the infestation.

Thus a human patient can receive a dose of at least 5 mg $kg^{-1}$ $day^{-1}$. For example, the dose can be 7.5 mg $kg^{-1}$ $day^{-1}$. Preferably the dose is at least 10 mg $kg^{-1}$ $day^{-1}$.

Higher doses, for example up to 20 mg $kg^{-1}$ $day^{-1}$ have been administered in cases of serious infestation. Typically an adult human patient weighs 70 kg.

In an adult human patient, a convenient dose which can be administered is 800 mg $day^{-1}$. This can be given in a single dose but preferably it is given in two 400 mg doses one in the morning and one in the evening.

The course of treatment is continued for at least a week, for example 2 or 3 weeks, but is preferably continued for a month. In cases of severe infestation, the treatment can continue for three months.

In order to use albendazole in this way, it is normally formulated in accordance with standard pharmaceutical practice as a pharmaceutical composition.

Albendazole is administered orally. For human use, orally administerable compositions include syrups, tablets, capsules and lozenges. A syrup formulation will generally consist of a suspension or solution of the compound in a liquid carrier for example, ethanol, glycerine or water with a flavouring or colouring agent. Where the composition is in the form of a tablet, any pharmaceutical carrier routinely used for preparing solid formulations may be employed.  Examples of such carriers include magnesium stearate, starch, lactose and sucrose.  Where the composition is in the form of a capsule, the solid in granular form optionally with a binding agent is encapsulated in an edible shell e.g. of gelatin.

Preferably the composition is in unit dose form, for example a tablet or capsule, so that the patient may administer to himself a single dose.

Each dosage unit can contain from 200 to 800 mg of albendazole.  Known albendazole compositions suitable for oral administration to man contain 200 mg of the compound per unit dose.  For the present invention, the unit dose preferably contains at least 400 mg.  Such compositions are novel.

Accordingly, in a further aspect, the invention provides a pharmaceutical composition suitable for oral administration ot man comprising albendazole in an amount such that a unit dose contains at least 400 mg of albendazole.

In particular the composition can contain 400 mg per unit dose or 800 mg per unit dose.

The compositions described above can be prepared by standard methods.

The following Examples illustrate the invention.

## EXAMPLES

### Example 1

Tablets were made from the following ingredients :-

|                              | mg/tablet |
|------------------------------|-----------|
| Albendazole                  | 400       |
| Lactose                      | 560.0     |
| Maize starch                 | 160.0     |
| Polyvinylpyrrolidone         | 20.0      |
| Sodium lauryl sulphate       | 2.0       |
| Purified water               | q.s.      |
| Sodium starch glycollate     | 80.0      |
| Microcrystalline cellulose   | 100.0     |
| Sodium saccharin             | 2.0       |
| Magnesium stearate           | 6.0       |

Albendazole, lactose and maize starch are mixed until homogeneous. The polyvinylpyrrolidone and sodium lauryl sulphate are added as a 12% w/v and a 1.2% w/v aqueous solution respectively with continuous mixing and mixing is continued until the mixture is homogeneous. The mass is then granulated in a mill, dried at 60°C. and screened to produce granules. The microcrystalline cellulose sodium starch glycollate, magnesium stearate and sodium saccharin are mixed and screened (the first mixture). This first mixture is then added to the granules and mixed until a homogeneous mixture (the second mixture) is obtained. The second mixture is compressed on punches and dies to form tablets.

A polymer aqueous film coating solution is made up containing methylcellulose - viscosity 5 cps (3.72 w/v ), methylcellulose - viscosity 15 cps (3.72 w/v) and purified water to 100.

A quantity of tablets are loaded into a conventional rotating-drum spray-coating machine and loose dust is removed by tumbling the tablets through a hot air (90°C) stream for a short period (ca 10 seconds). The tablets are then sprayed and dried alternately with the polymer aqueous film coating solution using an electronically timed spray gun which intermittently sprays the tumbling tablets with the solution. The period of spraying and subsequent drying periods were set so that the tablets were not visibly wet at any time. The spraying/drying cycle is continued until the tablets are coated.

The coated tablets are transferred to a canvas polishing pan, mixed with finely powdered canuba wax (250 microns) and rolled until a sheen develops.

Example 2

Tablets containing 200 mg of albendazole can be prepared using the same ingredients and the same method as described in Example 1 but by, compressing instead appropriately smaller quantities of the second mixture.

Example 3

A suspension is made from the following ingredients:-

|                                              |     g       |
| -------------------------------------------- | ----------- |
| Albendazole                                  | 4.0         |
| Veegum Regular                               | 0.65        |
| Sodium Carboxymethyl-Cellulose 7H3SF         | 0.42        |
| Glycerin                                     | 5.0         |
| Tween 80                                     | 0.4         |
| Span 20                                      | 0.27        |
| Potassium Sorbate                            | 0.15        |
| Benzoic Acid                                 | 0.10        |
| Silicone Antifoam Emulsion 1510              | 0.07        |
| Saccharin Sodium                             | 0.05        |
| Orange Flavour Firmenish 51914/T             | 0.165       |
| Purified water                               | to 100.0 ml |

The Tween 80 and Span 20 are added to part of the purified water (ca 30 ml) with stirring until a solution is obtained. The albendazole is added to this solution with stirring until it is completely dispersed (the first mixture). This first mixture is left to stand to deaerate for at least 3 hr.

The Veegum is added over ca 2 mins to another part (ca 30 ml) of the purified water with mixing in a high shear mixer. The mixing is continued for a further 30 mins and then the potassium sorbate, benzoic acid and saccharin sodium are added. Mixing is continued for a further 10 mins. The silicone antifoam emulsion is then added with mixing which is continued for 5 min to produce a uniform mixture (the second mixture).

The second mixture is pumped into the first mixture under its surface, while it is being stirred with a low shear stirrer. To this is added the orange flavour and substantially all the remaining water. The mixture is stirred until homogeneous and left to deaerate for at least 30 min (the third mixture).

The sodium carboxymethylcellulose is dispersed in the glycerin and this mixture is then added to the fourth mixture which is mixed in a low shear mixer for 5 mins. This final mixture is made up to 100 ml with the purified water.

Example 4

A 2% suspension can be prepared using the ingredients and method described in Example 3 substituting 2.0 g albendazole for 4.0 g.

Example 5

A suspension was made from the following ingredients:-

|                                           | g         |
|-------------------------------------------|-----------|
| Albendazole                               | 2.0       |
| Sodium Laurylsulphate                     | 0.1       |
| Glycerin                                  | 10.0      |
| Methyl Parahydroxybenzoate                | 0.2       |
| Propyl Parahydroxybenzoate                | 0.04      |
| Sodium Carboxymethyl-Cellulose 7MF        | 1.75      |
| Sodium Carboxymethyl-Cellulose 7HF        | 0.05      |
| Saccharin.Sodium                          | 0.05      |
| Orange Flavour 519.14/T                   | 0.165     |
| Purified Water                            | to 100 ml |

Albendazole (2.0 g) was dispersed in a solution of sodium laurylsulphate (0.1 g) in water (15 ml) (mixture A).

Sodium carboxymethyl-cellulose 7MF and 7HF in the quantities above were dissolved in half of the glycerin. The methyl and propyl parahydroxybenzoates were dissolved in the remaining glycerin, which had been heated (70°C) to aid solution. The two glycerin solutions were combined and diluted with water (70 ml) and the orange flavour was added to the dilute solution (solution B).

Mixture A and solution B were mixed and a solution of the saccharin in water (0.5 ml) was added. This mixture was then made up to 100 ml.

Example 6

A 41-year old female patient with a previous history of hydatid disease (two hepatic hydatid cysts were removed 10 years previously) presented complaining of epigastric pain and pain in the left chest and left shoulder. On examination the left upper quadrant of her abdomen was tender.

A complement fixation test was positive (1/52).

X-ray showed cysts in the left mediastinum, left lung and left chest wall. C.T. scan showed multiple cysts in the mediastinum, lung, chest wall, perineum and retro-peritoneum.

The patient was given albendazole (400 mg, p.o. twice a day; 10 mg $kg^{-1}$) for one month.

After one month of treatment the pain complained of had gone. X-ray showed that the cysts had disappeared from the mediastinum and lung.

C.T. scan showed that all the cysts were much smaller and most had disappeared.

The complement fixation test was (1/16).

Example 7

A 74-year old female patient presented complaining of pain in the right upper quadrant of her abdomen and was very short of breath.

C.T. scan showed a very large cyst on the right lobe of the liver projecting through the diaphragm.

The complement fixation test was positive (1/32).

The patient was given albendazole (200 mg, p.o. twice a day; 10 mg $kg^{-1}$).

After one month, the cyst had virtually disappeared. The complement fixation test was (1/16).

CLAIMS

1.    Albendazole for use by oral administration in the treatment of hydatid disease in man.

2.    Albendazole for use as claimed in claim 1 where the dose is at least 5 mg $kg^{-1}$ $day^{-1}$.

3.    Albendazole for use as claimed in claim 1 where the dose is 7.5 mg $kg^{-1}$ $day^{-1}$.

4.    Albendazole for use as claimed in claim 1 where the dose is 10 mg $kg^{-1}$ $day^{-1}$.

5.    Albendazole for use as claimed in claim 1 in human patients where the daily dose is 800 mg per day.

6.    A pharmaceutical composition suitable for oral administration to man containing albendazole in an amount such that a unit dose contains at least 400 mg of albendazole.